(19) Europäisches Patentamt
European Patent Office
Office européen des brevets

(11) Veröffentlichungsnummer: **0 288 898 B1**

(12) # EUROPÄISCHE PATENTSCHRIFT

(45) Veröffentlichungstag der Patentschrift: **14.08.91**

(51) Int. Cl.5: **C07D 403/04**, A61K 31/415, A61K 31/495

(21) Anmeldenummer: **88106355.6**

(22) Anmeldetag: **21.04.88**

(54) **Substituierte Chinoxalyl-imidazolidin-2,4-dione, Verfahren zu ihrer Herstellung, ihre Verwendung als Arzneimittel sowie pharmazeutische Präparate.**

(30) Priorität: **25.04.87 DE 3713872**

(43) Veröffentlichungstag der Anmeldung:
**02.11.88 Patentblatt 88/44**

(45) Bekanntmachung des Hinweises auf die Patenterteilung:
**14.08.91 Patentblatt 91/33**

(84) Benannte Vertragsstaaten:
**AT BE CH DE ES FR GB GR IT LI LU NL SE**

(56) Entgegenhaltungen:

**JOURNAL OF MEDICINAL CHEMISTRY, Band 28, Nr. 7, 1. Juli 1985, Seiten 841-849, American Chemical Society, Washington DC, US; P.F. KADOR et al.: "Aldose reductase inhibitors: A potential new class of agents for the pharmacological control of certain diabetic complications"**

(73) Patentinhaber: **HOECHST AKTIENGESELLSCHAFT**
**Postfach 80 03 20**
**W-6230 Frankfurt am Main 80(DE)**

(72) Erfinder: **Glombik, Heiner, Dr.**
**Südhang 16**
**W-6238 Hofheim am Taunus(DE)**
Erfinder: **Utz, Roland, Dr.**
**Sudetenstrasse 18**
**W-6101 Messel(DE)**
Erfinder: **Lang, Hans-Jochen, Dr.**
**Rüdesheimer Strasse 7**
**W-6238 Hofheim am Taunus(DE)**
Erfinder: **Geisen, Karl, Dr.**
**Jahnstrasse 43**
**W-6000 Frankfurt am Main(DE)**
Erfinder: **Beyhl, Friedrich Ernst, Dr.**
**Nonnbornstrasse 23**
**W-6233 Kelkheim (Taunus)(DE)**

EP 0 288 898 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Substituierte Chinoxalyl-imidazolidin-2,4-dione, Verfahren zu ihrer Herstellung, ihre Verwendung als Arzneimittel sowie pharmazeutische Präparate

Bei der Therapie des Diabetes mellitus werden bisher im wesentlichen Substanzen eingesetzt, die die Blutglucoseund Insulinspiegel normalisieren.

In jüngster Zeit wurden Verbindungen zur Beeinflussung von chronischen diabetischen Schäden und Spätkomplikationen beschrieben. Zu diesen gehören die diabetische Neuropathie, die Microangiopathie - primär als Retinopathie und als Nephropathie - sowie die Kataraktbildung. Hemmstoffe des Enzyms Aldose-Reduktase können neueren Forschungsergebnissen zufolge zur Behandlung dieser Krankheiten eingesetzt werden. Zu den am stärksten wirkenden bisher bekannten Aldose-Reduktase-Inhibitoren zählen spiroverknüpfte Imidazolidindione (P.F. Kador, J.H. Konoshita und N.E. Sharpless, J. Med. Chem. 28 [1985], 841).

Überraschenderweise wurde nun gefunden, daß geeignet substituierte Chinoxalyl-imidazolidindione die Aldose-Reduktase mit hoher Wirkstärke hemmen und daher zur Behandlung chronischer diabetischer Komplikationen verwendet werden können.

Die Erfindung betrifft daher 5-Chinoxalyl-imidazolidin-2,4-dione der Formel I

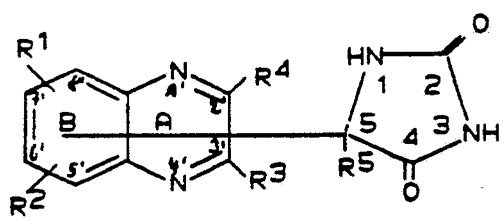

I

in welcher

- das Chinoxalin-Ringsystem über die Positionen 2', 3', 5', 6', 7' oder 8' mit den Imidazolidindion verbunden ist,
- $R^1$ und $R^2$ gleich oder verschieden sind und Wasserstoff, $(C_1-C_6)$-Alkyl, $(C_5-C_7)$-Cycloalkyl, Phenyl-$(C_1-C_4)$-alkyl, Napthyl-$(C_1-C_4-)$-alkyl, $(C_6-C_{12})$-Aryl, 5- bis 6-gliedriger Heteroarylring mit Schwefel, Sauerstoff oder 1 bis 2 Stickstoffatomen als Heteroatom(e), der gegebenenfalls benzoanneliert ist, $(C_1-C_4)$-Alkoxy, Halogen, Halogen-$(C_1-C_4)$-alkyl, Nitro, Amino oder Hydroxy bedeuten;
- $R^3$ und $R^4$ gleich oder verschieden sind und Wasserstoff, $(C_1-C_6)$-Alkyl, $(C_5-C_7)$-Cycloalkyl, Phenyl-$(C_1-C_4)$-alkyl, Naphthyl-$(C_1-C_4)$-alkyl, $(C_6-C_{12})$-Aryl, 5- bis 6-gliedriger Heteroarylring mit Schwefel, Sauerstoff oder 1 bis 2 Stickstoffatomen als Heteroatom(e), der gegebenenfalls benzoanneliert ist, Amino, Nitro oder Hydroxy bedeuten;
- $R^5$ ein Wasserstoffatom, $(C_1-C_6)$-Alkyl, $(C_5-C_7)$-Cycloalkyl, Phenyl-$(C_1-C_4)$-alkyl, Naphthyl-$(C_1-C_4)$-alkyl oder $(C_6-C_{12})$-Aryl bedeutet,
- $R^3$ und $R^5$ oder $R^4$ und $R^5$ falls Ring A mit Imidazolidindion substituiert ist, auch gemeinsam eine gegebenenfalls mit $(C_1-C_4)$-Alkyl substituierte $-(CH_2)_n$-Gruppe mit n = 2, 3 oder 4 bilden können, oder $R^3$ und $R^4$ falls Ring B mit Imidazolidindion substituiert ist, auch gemeinsam eine gegebenenfalls mit $(C_1-C_4)$-Alkyl substituierte $-(CH_2)_m$-Gruppe mit m = 3, 4 oder 5 bilden können,

sowie deren physiologisch verträgliche Salze.

Ist der Imidazolidindionrest mit Ring A verbunden, so ersetzt er einen der Reste $R^3$, $R^4$.

Bevorzugt sind Verbindungen der Formel I, worin

a) das Chinoxalin-Ringsystem über die Position 2' oder 3' mit dem Imidazolidindion verbunden ist und $R^1$ und $R^2$ in den Positionen 6' bzw. 7' stehen und Wasserstoff, $(C_1-C_4)$-Alkyl, Phenyl, $(C_1-C_4)$-Alkoxy, Halogen, Halogen-$(C_1-C_4)$-alkyl, Amino oder Hydroxy bedeuten, einer der Substituenten $R^3$ oder $R^4$ ein Wasserstoffatom, $(C_1-C_4)$-Alkyl, Phenyl, Pyridyl, Furyl, Thienyl oder Benzofuryl bedeutet und $R^5$ Wasserstoff oder $(C_1-C_4)$-Alkyl ist oder die Reste $R^3$ bzw. $R^4$ und $R^5$ gemeinsam eine gegebenenfalls mit $(C_1-C_4)$-Alkyl substituierte $-(CH_2)_n$-Gruppe mit n = 2, 3 oder 4 bilden,

b) das Chinoxalin-System über eine Position 5', 6' oder 7' mit dem Imidazolidindion verbunden ist, und

- $R^3$ und $R^4$ ein Wasserstoffatom, $(C_1-C_6)$-Alkyl, $(C_5-C_7)$-Cycloalkyl, Phenyl-$(C_1-C_2)$-alkyl, Phenyl, Pyridyl, Furyl, Thienyl oder Benzofuryl bedeuten, $R^1$ ein Wasserstoffatom ist und $R^2$ ein Wasserstoffatom, $(C_1-C_4)$-Alkyl oder Phenyl bedeuten und $R^5$ Wasserstoff oder $(C_1-C_4)$-Alkyl ist, oder
- die Reste $R^3$ und $R^4$ gemeinsam eine gegebenenfalls mit $(C_1-C_4)$-Alkyl substituierte $-(CH_2)_m$-Gruppe mit m = 3, 4 oder 5 bilden,

sowie deren physiologisch verträgliche Salze.

Besonders bevorzugt sind Verbindungen der Formel I, worin

- das Chinoxalin-Ringsystem im Fall a) über die Position 2′ oder 3′ oder im Fall b) über die Position 6′ oder 7′ an die Imidazolidindion-Partialstruktur gebunden ist;
- im Fall a) die Substituenten $R^1$, $R^2$ in den Postionen 6′ und 7′ stehen und ein Wasserstoffatom, $(C_1-C_4)$-Alkyl, $(C_1-C_4)$-Alkoxy, Fluor, Trifluormethyl, Amino oder Hydroxy bedeuten und $R^3$ oder $R^4$ ein Wasserstoffatom, $(C_1-C_4)$-Alkyl oder Phenyl bedeutet und $R^5$ Wasserstoff oder Methyl ist oder die Reste $R^3$ und $R^5$ oder $R^4$ und $R^5$ gemeinsam eine gegebenenfalls mit $(C_1-C_4)$-Alkyl substituierte $-(CH_2)_3$-Gruppe bilden, so daß ein anellierter Sechsring vorliegt;
- im Fall b) die Substituenten $R^3$, $R^4$ ein Wasserstoffatom, $(C_1-C_6-)$-Alkyl, $(C_5-C_7)$-Cycloalkyl, Benzyl, Phenyl, Furyl oder Thienyl bedeuten, $R^1$ Wasserstoff ist und $R^2$ ein Wasserstoffatom, $(C_1-C_4)$-Alkyl oder Phenyl bedeutet und $R^5$ Wasserstoff oder Methyl ist oder die Reste $R^3$ und $R^4$ gemeinsam eine $-(CH_2)_4$-Gruppe bilden, so daß ein an das Chinoxalin-System anellierter Sechsring vorliegt,

sowie deren physiologisch verträgliche Salze.

Alkyl kann geradkettig oder verzweigt sein. Unter $(C_6-C_{12})$-Aryl versteht man beispielsweise Naphthyl oder Biphenyl, insbesondere aber Phenyl.

Die Verbindungen der Formel I können sowohl in optisch inaktiver als auch in enantiomerenreiner Form vorliegen.

Die Erfindung betrifft weiterhin ein Verfahren zur Herstellung von Verbindungen der Formel I, dadurch gekennzeichnet, daß man

a) eine Carbonylverbindung der Formel II

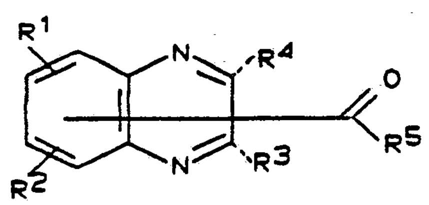

II

worin $R^1$, $R^2$, $R^3$, $R^4$ und $R^5$ die zu Formel I angegebenen Bedeutungen haben mit Cyaniden, vorzugsweise mit Alkali-und Erdalkalimetallcyaniden und Ammoniumcarbonat zu Verbindungen der Formel I umsetzt oder

b) eine Verbindung der Formel III

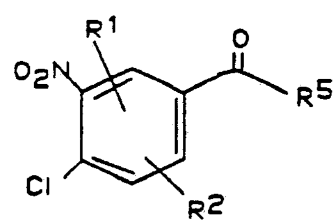

III

worin $R^1$ Wasserstoff ist und $R^2$ und $R^5$ die zu Formel I angegebenen Bedeutungen haben mit Benzylamin

( 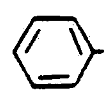 $CH_2-NH_2$)

zu einer Verbindung der Formel IV

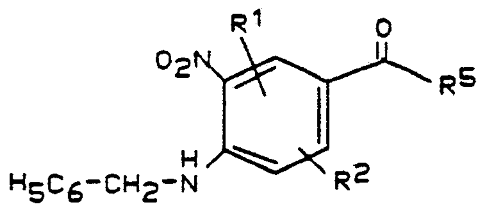

IV

umsetzt, die erhaltene Verbindung der Formel IV mit einem Cyanid, vorzugsweise einem Alkali- oder Erdalkalimetallcyanid, und Ammoniumcarbonat zu einer Verbindung der Formel V

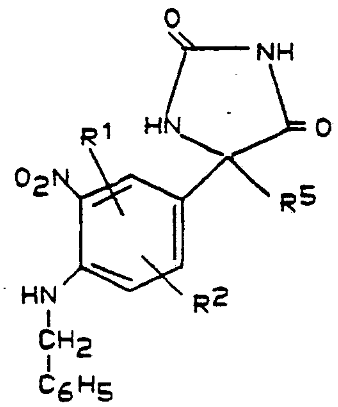

V

umsetzt, die erhaltene Verbindung in Gegenwart eines Katalysators zu einer Verbindung der Formel VI

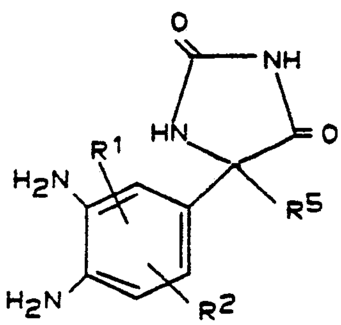

VI

reduziert und anschließend mit einer Dicarbonylverbindung VII

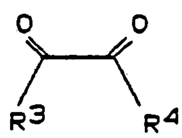

VII

zu einer Verbindung der Formel I

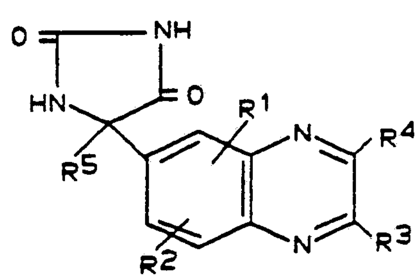

I

worin $R^1$ Wasserstoff ist $R^2$, $R^3$, $R^4$, $R^5$ die angegebenen Bedeutungen haben, umsetzt und die Reaktionsprodukte gegebenenfalls mit Basen in physiologisch verträgliche Salze überführt.

Bei der Synthese gemäß Verfahren a) arbeitet man vorteilhaft bei Normaldruck oder im Autoklaven und Temperaturen zwischen 50 °C und 160 °C mit Reaktionszeiten von 1 bis 80 Stunden.

Obwohl die relative Menge der Reagenzien in gewissen Grenzen variieren kann, wird vorzugsweise mindestens ein geringer Überschuß des Cyanids und des Ammoniumcarbonats, bezogen auf die Carbonylkomponente eingesetzt, um eine gute Ausbeute zu erzielen.

Die Carbonylverbindung der Formel II wird zweckmäßigerweise in geeigneten Lösungsmitteln wie Ethern (z.B. Dioxan, Dimethoxyethan), Alkoholen (z.B. Ethanol, Methanol, Methoxyethanol) oder Gemischen dieser Lösungsmittel mit Wasser jeweils mit einen Überschuß an Cyanid (1,5 bis 3 Äquivalente) und einem größeren Überschuß an Ammoniumcarbonat (2 bis 5 Äquivalente) umgesetzt.

Die Carbonyl-Chinoxaline der Formel II können, soweit sie nicht in der Literatur beschriebenen wurden (vgl. z.B. J. Francis et al., Biochem. J. 63 (1956), 455), durch Kondensation von geeignet substituierten Phenylendiaminen mit 1,2-Dicarbonylverbindungen, mit 1,2,3-Tricarbonylverbindungen oder mit deren Syntheseäquivalenten hergestellt werden.

Verfahren b) stellt eine in einigen Fällen günstigere Variante zur Herstellung von Verbindungn der Formel I dar. Es besteht darin, daß man zunächst aus bekannten Vorstufen der Formel III (Lit.: z.B. F. Mayer et. al., Chem. Ber. 56 (1932) 1295) das Imidazolidin-Ringsystem aufbaut und anschließend mit einer geeigneten Dicarbonylkomponente (Formel VII, z.B. Hexan-3,4-dion) zum Chinoxalin kondensiert.

Basen, die erfindungsgemäß als Reaktionspartner zur Herstellung der pharmakologisch verträglichen Salze verwendet werden, sind solche, die nicht-toxische Salze mit den sauren Imidazolidindionen der Formel I bilden. Dabei müssen die Kationen der Salze nicht-toxisch über dem weiten Bereich der verabreichten Dosen sein. Beispiele sind Natrium-, Kalium-, Calcium- und Magnesium-Ionen.

Die Salze können hergestellt werden, indem man z.B. die Imidazolidindione der Formel I mit einer wäßrigen Lösung des gewünschten pharmakologisch verträglichen Kations behandelt und die erhaltene Lösung - vorzugsweise unter vermindertem Druck - bis zur Trockne eindampft.

Die erfindungsgemäßen Verbindungen der Formel I haben wertvolle pharmakologische Eigenschaften und können in der Therapie als Aldose-Reduktase-Inhibitoren zur Verhinderung chronischer diabetischer Komplikationen eingesetzt werden. Dazu gehören z.B. Katarakt und Retinopathie, periphere Neuropathie, Nephropathie, Microangiopathie und verzögerte Cornea-Reepithelialisierung (P.F. Kador et al., J. Med. Chem. 28, [1985] 841).

Die Substanzen besitzen eine sehr hohe Aldose-Reduktase-Hemmwirkung.

Die Aktivität der erfindungsgemäßen Verbindungen als Wirkstoffe zur Behandlung chronischer diabetischer Komplikationen wird durch ihre Eigenschaft bestimmt, einen oder mehrere der folgenden biologischen und/oder pharmakologischen Tests erfolgreich zu erfüllen:

1. in vitro-Hemmung der Enzymaktivität von abgetrennter Aldose-Reduktase;
2. in vitro-Hemmung der Sorbitol-Akkumulation in Erythrocyten, in der Linse oder im Ischiasnerv normaler oder Streptozotocin-diabetischer Ratten;
3. Senkung erhöhter Sorbitolspiegel an Streptozotocin-diabetischen Ratten in vivo;
4. Verhinderung oder Hemmung der Galactit-Bildung in den Linsen galactosämischer Ratten oder Hunde;
5. Verhinderung der Katarakt-Bildung und Verminderung der Linsentrübung bei galactosämischen und diabetischen Ratten oder Hunden;
6. Normalisierung der Nervenleitungsgeschwindigkeit diabetischer Versuchstiere;
7. Verhinderung der Basalmembranverdickung diabetischer Versuchstiere.

Die Testmethoden sind im Prinzip in der Literatur beschrieben.

Für einige erfindungsgemäße Verbindungen konnte in mehreren Tests eine hohe Aktivität beobachtet werden.

**Test Nr. 1**

In vitro-Hemmung der Enzymaktivität
(Rattenleberenzympräparat)

Die Hemmwerte auf die Aldose-Reduktase sind für einige erfindungsgemäße Verbindungen in Tabelle 1 zusaengestellt ($IC_{50}$ [M] bedeutet molare Konzentration der Verbindung, die für eine 50 %ige Hemmung erforderlich ist).

**Tabelle 1**

| Verbindung gemäß Beispiel Nr. | $IC_{50}$ [M] |
|---|---|
| 2 | $1,2x10^{-6}$ |
| 18 | $2,8x10^{-4}$ |
| 19 | $7,3x10^{-6}$ |
| 20 | $3,6x10^{-7}$ |
| 21 | $1,5x10^{-4}$ |
| 22 | $4,4x10^{-4}$ |
| 23 | $1,4x10^{-5}$ |
| 26 | $2,8x10^{-4}$ |
| 33 | $2,7x10^{-9}$ |

**Test Nr. 2**
In vitro-Hemmung der Sorbitolakkumulation
(Gewebe normaler Ratten)

Bei einer Konzentration von 3 μMol/1 wurden für einige der erfindinngsgemäßen Verbindungen die in Tabelle 2 zusammengestellten Hemmwerte ermittelt.

**Tabelle 2**

| Verbindung gemäß Beispiel Nr. | Nerv [%] | Erythrozyten [%] |
|---|---|---|
| 2 | -30 | |
| 18 | -39 | -10 |
| 20 | | -29 |
| 21 | -38 | -26 |
| 23 | -65 | -21 |
| 33 | -30 | -26 |

**Test Nr. 3**
Senkung erhöhter Sorbitolspiegel

Die orale Verabreichung der beispielhaft in der folgenden Tabelle 3 genannten Substanzen in ®Tylose-Suspension (=Suspension eines wasserlöslichen Celluloseethers) an Streptozotocin-diabetischen Ratten führt im Vergleich zu unbehandelten Kontrolltieren zu einer starken Absenkung der Sorbitolakkumulation in Erythrocyten und im Nervengewebe.

**Tabelle 3**

| Verbindung gemäß Beispiel Nr. | Dosis | Sorbitolgehalt nach 5 Std. in Erythrozyten | Nervengewebe |
|---|---|---|---|
| 2 | 25 mg/kg | - 57 % | - 22 % |
| 23 | 25 mg/kg | - 72 % | - 83 % |
| 25 | 25 mg/kg | - 66 % | - 70 % |

Aufgrund ihrer pharmakologischen Aktivitäten können die erfindungsgemäßen Verbindungen der Formel I oder ihre Salze als Arzneimittel, insbesondere zur Hemmung der Aldose Reduktase, verwendet werden. Die Erfindung betrifft daher auch die Verwendung der Verbindungen der Formel 1 als Arzneimittel.

Die neuen Verbindungen und deren Salze können entweder allein oder gemeinsam mit physiologisch verträglichen Hilfs- oder Trägerstoffen als Arzneimittel angewandt werden. Sie können zu diesem Zweck enteral in Dosen von [0,05 bis 5,0] mg/(kgTag), vorzugsweise [0,5 bis 2,5] mg/(kgTag) oder parenteral in Dosen von [0,005 bis 0,5] mg/(kgTag), vorzugsweise [0,05 bis 0,25] mg/(kgTag) appliziert werden.

Die Dosierung kann in schweren Fällen auch erhöht werden, in vielen Fällen genügen jedoch schon geringere Dosen.

Zusätzlich können die Substanzen der Formel I örtlich über eine geeignete, tropfenweise am Auge verwendbare ophtalmische Lösung für die o.g. Zwecke eingesetzt werden, soweit sie das Auge betreffen.

Die Erfindung betrifft daher auch pharmazeutische Präparate, die eine Verbindung der Formel I oder deren Salz in einem physiologisch annehmbaren Träger enthalten sowie Verfahren zur Herstellung dieser Präparate, die dadurch gekennzeichnet sind, daß die genannten Substanzen zusammen mit dem Träger sowie auch Hilfs- oder Zusatzstoffen in eine geeignete Darreichungsform bringt.

Für die orale Anwendungsform werden die Verbindungen der Formel I mit den dafür geeigneten Substanzen vermischt und durch übliche Methoden in geeignete Darreichungsformen gebracht, wie z.B Tabletten, Steckkapseln, wäßrige alkoholische oder ölige Suspensionen oder wäßrige alkoholische oder ölige Lösungen. Als inerte Träger können beispielsweise Magnesiumcarbonat, Milchzucker oder Maisstärke verwendet werden. Für Tabletten kann die Zubereitung als Trocken- oder Feuchtgranulat erfolgen. Als ölige Trägerstoffe oder Lösungsmittel kommen pflanzliche oder mineralische Öle in Betracht, wie z.B. Sonnenblumenöl, Olivenöl oder Paraffinöl.

Zur parenteralen Applikation werden die aktiven Verbindungen oder deren physiologisch verträgliche Salze mit organischen oder anorganischen Basen wie z.B. Triethanolamin, Cyclohexylamin, Natrium- oder Kaliumhydroxid in dafür üblichen Substanzen suspendiert oder gelöst. Als Lösungsmittel für die intravenöse Anwendung kommen beispielsweise Wasser oder physiologische Kochsalzlösung in Betracht, die mit weiteren Zusätzen wie Alkohol (z.B. Propandiol, Glycerin) und Zuckerlösungen (z.B. Glucose oder Mannit) versehen sein können.

Die nachfolgenden Beispiele dienen zur Erläuterung der vorliegenden Erfindung, ohne diese auf die hier stellvertretend genannten Substanzen zu beschränken.

**Beispiel 1**

**5-(2′,3′-Dimethyl-chinoxalin-6′-yl)-5-methyl-imadazolidin-2,4-dion**

Man erhitzt eine Mischung aus 3,67 g (18,3 mmol) 2,3-Dimethyl-6-acetylchinoxalin, 1,79 g (27,5 mmol) Kaliumcyanid und 7,05 g (73, 3 mmol) Ammoniumcarbonat in 20 ml Ethanol/Wasser = 3 : 1 im Autoklaven 10 Std. auf 90 °C. Nach Abkühlung engt man ein, verdünnt mit Wasser und neutralisiert unter Rühren und Eiskühlung mit halbkonz. Salzsäure. Der ausgefallene Feststoff wird abgetrennt, in Methanol/Wasser gelöst, die Lösung mit Aktivkohle behandelt, abgesaugt und eingeengt. Das Produkt kristallisiert nach Zugabe von Diethylether aus.
Schmp.: 198 - 200 °C.

**Beispiel 2**

**5-(2',3'-Diethyl-chinoxalin-6'-yl)-5-methyl-imidazolidin-2,4-dion**

Eine Mischung aus 11,9 g (0,05 mol) 2,3-Diethyl-6-acetylchinoxalin, 4,90 g (0,075 mol) Kaliumcyanid und 19,2 g (0,20 mol) Ammoniumcarbonat werden in 100 ml Ethanol/Wasser = 3 : 1 im Autoklaven 10 Std. auf 90° C erhitzt. Nach Abkühlung bringt man den gebildeten Niederschlag mit etwas Wasser in Lösung und dampft den Alkohol i. Vak. weitgehend ab. Der Niederschlag wird abgesaugt, mit Wasser gewaschen und i. Vak. über Phosphorpentoxid getrocknet.
Schmp.: 212 - 213° C.

**Beispiel 3**

**5-(2'-3'-Di-n-pentyl-chinoxalin-6'-yl)-5-methyl-imidazolidin-2,4-dion**

Entsprechend Beispiel 2 aus 6-Acetyl-2,3-di-n-pentylchinoxalin.
Schmp.: 153 - 155° C.

**Beispiel 4**

**5-(2',3'-Diisopropyl-chinoxalin-6'-yl)-5-methyl-imidazolidin-2,4-dion**

Entsprechend Beispiel 2 aus 6-Acetyl-2,3-diisopropylchinoxalin.
Schmp.: 210 - 212° C.

**Beispiel 5**

**5-(2',3'-Diethyl-7'-methyl-chinoxalin-6'-yl)-5-methyl-imidazolidin-2,4-dion**

Analog Beispiel 2 aus 6-Acetyl-2,3-diethyl-7-methylchinoxalin.
Schmp.: 228 - 229° C.

**Beispiel 6**

**5-Methyl-5-(1',2',3',4'-tetrahydrophenazin-7'-yl)-imidazolidin-2,4-dion**

Analog Beispiel 2 aus 7-Acetyl-1,2,3,4-tetrahydrophenazin.
Schmp.: 196 - 202° C.

**Beispiel 7**

**5-[2',3'-(Difuran-2''-yl)-chinoxalin-6'-yl]-5-methyl-imidazolidin-2,4-dion**

Entsprechend Beispiel 2 aus 6-Acetyl-2,3-(difuran-2'-yl)-chinoxalin.
Schmp.: 265° C (Zers.).

**Beispiel 8**

**5-(2',3'-Diphenyl-chinoxalin-6'-yl)-5-methyl-imidazolidin-2,4-dion**

Aus 6-Acetyl-2,3-diphenyl-chinoxalin analog Beispiel 2.
Schmp.: 316° C (Zers.).

**Beispiel 9**

**5-Methyl-5-(2'-phenyl-chinoxalin-6'-yl)-imidazolidin-2,4-dion**

Analog Beispiel 2 aus 6-Acetyl-2-phenyl-chinoxalin.
Schmp.: 269 - 271° C.

**Beispiel 10**

**5-(2',3'-Diethyl-chinoxalin-5'-yl)-5-methyl-imidazolidin-2,4-dion**

Analog Beispiel 2 aus 5-Acetyl-2,3-diethyl-chinxalin.
Schmp. 216 - 217° C.

**Beispiel 11**

**5-Methyl-5-(2',3'-pentamethylen-chinoxalin-5'-yl)-imidazolidin-2,4-dion**

Aus 5-Acetyl-2,3-pentamethylen-chinoxalin entsprechend Beispiel 2.
Schmp. 296 - 298° C.

**Beispiel 12**

**5-(2',3'-Diethyl-7'-methyl-chinoxalin-6'-yl)-5-propyl-imidazolidin-2,4-dion**

Entsprechend Beispiel 2 aus 6-Butyryl-2,3-diethyl-7-methyl-chinoxalin.
Schmp.: 217 - 219° C.

**Beispiel 13**

**a) 5-Methyl-5-(2'-thienyl-chinoxalin-6'-yl)-imidazolidin-2,4-dion und**
**b) 5-Methyl-5-(3'-thienyl-chinoxalin-6'-yl)-imidazolidin-2,4-dion**

Entsprechend Beispiel 2 aus 6-Acetyl-2/3-thienyl-chinoxalin erhält man nach Kristallisation zwei Produkte mit den Schmelzpunkten 273 - 274° C und 256 - 258° C; die eindeutige Isomerenzuordnung ist mit den verfügbaren spektroskopischen Methoden z. Z. nicht möglich.

**Beispiel 15**

**5-(2',3'-Diethyl-chinoxalin-6'-yl)-5-phenyl-imidazolidin-2,4-dion**

Eine Mischung aus 14,5 g (0,05 mol) 6-Benzoyl-2,3-diethyl-chinoxalin, 4.9 g (0,075 mol) Kaliumcyanid und 20 g (0,208 mol) Ammoniumcarbonat in 100 ml Ethanol/Wasser (7 : 3) wird in Autoklaven 15 Std. auf 110° C erhitzt. Nach dem Abkühlen filtriert man von unlöslichen Bestandteilen ab und dampft das Filtrat i. Vak. ein. Der Rückstand wird mit ca. 300 ml 1N Natronlauge aufgenommen, mit Aktivkohle behandelt, die wäßrige Lösung filtriert und dreimal mit je 120 ml Ether ausgeschüttelt. Die wäßrige Phase wird nochmals mit Aktivkohle behandelt und filtriert. Man neutralisiert mit halbkonz. Salzsäure, saugt den ausgefallenen Feststoff ab und wäscht gut mit Wasser nach. Die Substanz wird aus Ethanol/Wasser umkristallisiert.
Schmp.: 231 - 232° C.

**Beispiel 16**

**5-(2',3'-Dimethyl-chinoxalin-6'-yl)-5-phenyl-imidazolidin-2,4-dion**

Entsprechend Beispiel 15 aus 6-Benzoyl-2,3-dimethyl-chinoxalin.
Schmp.: 267 - 269° C.

**Beispiel 17**

**5-(Chinoxalin-6'-yl)-5-methyl-imidazolidin-2,4-dion**

**a) 4-Benzylamino-3-nitro-acetophenon**

29,9 g (0,15 mol) 4-Chlor-3-nitro-acetophenon werden in 90 ml Benzylamin gelöst und ca. 20 min auf 90° erhitzt. Nach Ende der Reaktion (Dünnschichtchromatogramm) wird die gelbbraune Lösung unter Rühren und Eiskühlung mit halbkonz. Salzsäure angesäuert und das ausgefallene Produkt in Ethylacetat aufgenommen. Die wäßrige Phase extrahiert man mehrfach mit Ethylacetat, wäscht die vereinigten

organischen Phasen mit Wasser und gesättigter Kochsalzlösung, trocknet über Natriumsulfat und dampft i. Vak. ein. Der Rückstand wird aus Methanol/Wasser/Dioxan umkristallisiert.
Schmp. 103 - 107° C.

**b) 5-(4′-Benzylamino-3′-nitro-phenyl)-5-methyl-imidazolidin-2,4-dion**

40,5 g (0,15 mol) 4-Benzylamino-3-nitro-acetophenon werden in einer Mischung aus 170 ml Ethanol, 60 ml Wasser, 14,6 g Kaliumcyanid und 58 g Ammoniumcarbonat im Autoklaven 18 Std. auf 100° C erhitzt.
Man dampft den Alkohol i. Vak. weitgehend ab, verdünnt mit Wasser, saugt ab und wäscht mit Wasser nach. Das Produkt wird aus Ethanol/Wasser unter Aktivkohlezusatz umkristallisiert.
Schmp.: 190 - 192° C.

**c)**

Eine Lösung von 11,4 g (33,5 mmol) 5-(4′-Benzylamino-3′-nitro-phenyl)-5-methyl-imidazolidin-2,4-dion in 350 ml Methanol wird bei Raumtemperatur in Gegenwart von 7,0 g Palladium/Kohle (5 %) bis zum Ende der Wasserstoffaufnahme hydriert. Man filtriert vom Katalysator ab, wäscht gut mit Methanol nach und dampft i. Vak. ein.

2,2 g der so gewonnenen Substanz werden in 24 ml Methanol/Dioxan (5 : 1) gelöst, mit 1,5 ml einer 40 %igen wäßrigen Glyoxallösung versetzt und 1 Std. auf 45 bis 50° C erwärmt. Nach beendeter Reaktion (Dünnschichtchromatogramm) dampft man das Lösungsmittel i. Vak. ab, löst den Rückstand in 1N Natronlauge, behandelt mit Aktivkohle, säuert mit 2N Salzsäure an und extrahiert dreimal mit Essigester. Die vereinigten organischen Phasen werden mit Wasser und gesättigter Kochsalzlösung gewaschen, über Natriumsulfat getrocknet und i. Vak. eingeengt. Den Rückstand kristallisiert man aus Aceton/Diisopropylether um.
Schmp.: 126 - 129° C.

## Beispiel 18

### 5-(Chinoxalin-2′-yl)-5-methyl-imidazolidin-2,4-dion

4,9 g (28, 5 mmol) 2-Acetyl-chinoxalin, 3,88 g (60 mmol) Kaliumcyanid und 13,62 g (0,14 mol) Ammoniumcarbonat werden in 60 ml Ethanol/Wasser 4 : 1 suspendiert und im Autoklaven 5 Std. bei 140° C gerührt. Nach dem Abkühlen versetzt man bis zur Lösung des Rückstands mit Wasser und säuert mit halbkonz. Salzsäure unter Eiskühlung vorsichtig an (pH = 5 - 6). Der gebildete Niederschlag wird abgesaugt, mit Wasser nachgewaschen und aus Methanol umkristallisiert.
Schmp.: 229° C.

## Beispiel 19

### 5-(6′,7′-Dimethyl-chinoxalin-2′-yl)-5-methyl-imidazolidin-2,4-dion

Entsprechend Beispiel 18 aus 2-Acetyl-6,7-dimethyl-chinoxalin, 8 Std. bei 135° C im Autoklaven.
Schmp.: 220 C.

## Beispiel 20

### 5-Methyl-5-(3′,6′,7′-trimethyl-chinoxalin-2′-yl)-imidazolidin-2,4-dion-hydrat

Entsprechend Beispiel 18 aus 2-Acetyl-3,6,7-trimethyl-chinoxalin, 8 Std. bei 140° C im Autoklaven.
Schmp.: 145 - 147° C (Zers.).

## Beispiel 21

### 5-(6′,7′-Dimethoxy-chinoxalin-2′-yl)-5-methyl-imidazolidin-2,4-dion

Analog Beispiel 18 aus 2-Acetyl-4,7-dimethyl-chinoxalin, 8 Std. bei 140° C, Umkristallisation aus Ethanol/Methylglykol.
Schmp.: 290° C.

## Beispiel 22

**5-Methyl-5-(3′-phenyl-chinoxalin-2′-yl)-imidazolidin-2,4-dion**

Entsprechend Beispiel 18 aus 2-Acetyl-3-phenyl-chinoxalin, 12 Std. bei 140 °C im Autoklaven.
Schmp.: 238 °C (Zers.).

**Beispiel 23**

**5-Methyl-5-(3′-methyl-chinoxalin-2′-yl)-imidazolidin-2,4-dion**

25 g (0,134 mol) 2-Acetyl-3-methyl-chinoxalin, 17,5 g (0,268 mol) Kaliumcyanid und 64,4 g (0,67 mol) Ammoniumcarbonat werden in 445 ml Ethanol/Wasser 1 : 1 gelöst und 72 Std. bei 60 °C gerührt. Nach beendeter Reaktion säuert man unter Eiskühlung vorsichtig mit halbkonz. Salzsäure an, läßt Nachrühren und saugt den gebildeten Niederschlag ab. Man wäscht mit Wasser nach, nimmt den Rückstand in 1N Natronlauge auf und schüttelt mit Ether aus. Durch Ansäuern mit halbkonz. Salzsäure unter Eiskühlung wird das Produkt freigesetzt. Man saugt ab, wäscht mit Wasser nach und kristallisiert aus Ethanol oder Methanol/Essigester 1 : 1 um.
Schmp. 225-227 °C.

**Beispiel 24**

**5-Methyl-5-(3′-methyl-6′/7′-trifluormethyl-chinoxalin-2′-yl)-imidazolidin-2,4-dion**

Analog Beispiel 23 aus 2-Acetyl-6/7-trifluormethyl-chinoxalin. Umkristallisation aus Essigester/Hexan. Schmp.: 217 °C, einheitliches Produkt nach Hochdruckflüssigkeitschromatogramm; die genaue Zuordnung der Substitution in 6- oder 7-Position ist mit den verfügbaren Methoden z. Z. nicht möglich.

**Beispiel 25**

**5-(6′/7′-Fluor-chinoxalin-2′-yl)-5-methyl-imidazolidin-2,4-dion**

Entsprechend Beispiel 18 aus 2-Acetyl-6/7-fluor-chinoxalin.
Schmp.: 221 °C.

**Beispiel 26**

**5-(6′/7′-Fluor-3′-methyl-chinoxalin-2′-yl)-5-methyl-imidazolidin-2,4-dion**

Analog Beispiel 24 aus 2-Acetyl-6/7-fluor-chinoxalin.
Schmp.: 271 °C.

**Beispiel 27**

**5-(6′/7′-Amino-3′-methyl-chinoxalin-2′-yl)-5-methyl-imidazolidin-2,4-dion**

Entsprechend Beispiel 24 aus 2-Acetyl-6/7-amino-chinoxalin.
Schmp.: 260 - 263 °C.

**Beispiel 28**

**5-(6′/7′-Hydroxy-3′-methyl-chinoxalin-2′-yl)-5-methyl-imidazolidin-2,4-dion**

Entsprechend Beispiel 24 aus 2-Acetyl-6/7-phenylsulfonyloxy-chinoxalin;
Schmp.: 268 - 271 °C.

**Beispiel 29**

**Spiro[imidazolidin-5,1′-(1′,2′,3′,4′-tetrahydro-phenazin)]-2,4-dion**

Entsprechend Beispiel 23 aus 1,2,3,4-Tetrahydro-phenazin-1-on.
Schmp.: 295° C (Zers.).

**Beispiel 30**

**Spiro[imidazolidin-5,1'-(3',3'-dimethyl-1',2',3',4'-tetrahydro-phenazin)]-2,4-dion**

**Entsprechend Beispiel 18 aus 3,3-Dimethyl-1,2,3,4-tetrahydro-phenazin-1-on.**
**Schmp.: 281°C.**

**Beispiel 31**

**Spiro[imidazolidin-5,1'-(8'fluor-3',3'-dimethyl,1',2',3',4'-tetrahydro-phenazin)]-2,4-dion**

Aus 8-Fluor-3,3-dimethyl-1,2,3,4-tetrahydro-phenazin-1-on analog Beispiel 18.
Schmp.: 292° C.

**Beispiel 32**

**(-)-5-Methyl-5-(3'-methyl-chinoxalin-2'-yl)-imidazolidin-2,4-dion**

10 g (39 mmol) (+/-)-5-Methyl-5-(3'-methyl-chinoxalin-2'-yl)-imidazolidin-2,4-dion und 16,8 (39 mmol) Brucindihydrat werden in 75 ml absolutem Ethanol in der Siedehitze gelöst. Die Lösung wird der langsamen Abkühlung überlassen. Das auskristallisierte Brucinsalz wird abgesaugt, i. Vak. getrocknet und mehrfach erneut aus ca. gleichem Lösungsmittelvolumen unter langsamer Abkühlung umkristallisiert, bis die Menge des trockenen Rückstands ungefähr 1/5 bis 1/4 der eingesetzten Substanzmenge entspricht. Das so erhaltene Salz kann durch Säulenfiltration über Kieselgel mit Ethylacetat/Methanol (1 : 1) gespalten werden. Durch Verwendung von Methanol/Triethylamin wird Brucin zurückgewonnen.

Die Titelverbindung wird durch fraktionierende Kristallisation aus Ethylacetat/Methanol (1:1) gewonnen; Schmp.: 203 - 204° C, $[\alpha]_D$ = -52,4° C.

**Beispiel 33**

**(+)-5-Methyl-5-(3'-methyl-chonoxalin-2'-yl)-imidazolidin-2,4-dion**

Die Mutterlauge der ersten Kristallisation aus Beispiel 32 wird i. Vak. vom Lösungsmittel befreit. Der Rückstand wird in ca. 50 ml abs. Ethanol erwärmt, die Lösung mit Impfkristallen des Salzes aus Brucin und linksdrehendem Enantiomer versetzt und langsam abgekühlt.

Das auskristallisierte Salz wird abgesaugt und die Mutterlauge eingedampft. Dieses Verfahren wird solange wiederholt, bis die Menge des trockenen Rückstands nach dem Eindampfen ca. 1/5 bis 1/4 der ursprünglich in die Beispielreaktion 32 eingesetzten Substanzmenge entspricht.

Danach wird das erhaltene Salz analog zu Beispiel 32 gespalten. Man erhält die Titelverbindung durch fraktionierende Kristallisation des Rohprodukts aus Ethylacetat/Methanol (1 : 1).
Schmp.: 203 - 204° C, $[\alpha]_D$ = +52,5° C.

**Patentansprüche**
**Patentansprüche für folgende Vertragsstaaten : AT, BE, CH, DE, FR, GB, IT, LI, LU, NL, SE**

1. 5-Chinoxalyl-imidazolidin-2,4-dione der Formel I

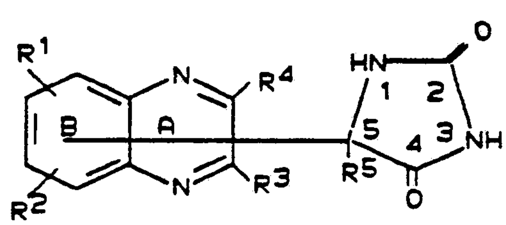

I

in welcher

- das Chinoxalin-Ringsystem über die Positionen 2', 3', 5', 6', 7', oder 8' mit dem Imidazolidindion verbunden ist,
- $R^1$ und $R^2$ gleich oder verschieden sind und Wasserstoff, ($C_1$-$C_6$)-Alkyl, ($C_5$-$C_7$)-Cycloalkyl, Phenyl-($C_1$-$C_4$)-alkyl, Naphthyl-($C_1$-$C_4$)-alkyl, ($C_6$-$C_{12}$)-Aryl, 5- bis 6-gliedriger Heteroarylring mit Schwefel, Sauerstoff oder 1 bis 2 Stickstoffatomen als Heteroatom(e), der gegebenenfalls benzo-anneliert ist, ($C_1$-$C_4$)-Alkoxy, Halogen, Halogen-($C_1$-$C_4$)-alkyl, Nitro, Amino oder Hydroxy bedeuten;
- $R^3$ und $R^4$ gleich oder verschieden sind und Wasserstoff, ($C_1$-$C_6$)-Alkyl, ($C_5$-$C_7$)-Cycloalkyl, Phenyl-($C_1$-$C_4$)-alkyl, Naphthyl-($C_1$-$C_4$)-alkyl, ($C_6$-$C_{12}$)-Aryl, 5- bis 6-gliedriger Heteroarylring mit Schwefel, Sauerstoff oder 1 bis 2 Stickstoffatomen als Heteroatom(e), der gegebenenfalls benzoanneliert ist, Amino, Nitro oder Hydroxy bedeuten;
- $R^5$ ein Wasserstoffatom, ($C_1$-$C_6$)-Alkyl, ($C_5$-$C_7$)-Cycloalkyl, Phenyl-($C_1$-$C_4$)-alkyl, Naphthyl-($C_1$-$C_4$)-alkyl oder ($C_6$-$C_{12}$)-Aryl bedeutet,
- $R^3$ und $R^5$ oder $R^4$ und $R^5$ falls Ring A mit Imidazolidindion substituiert ist, auch gemeinsam eine gegebenenfalls mit ($C_1$-$C_4$)-Alkyl substituierte $-(CH_2)_n$-Gruppe mit n = 2, 3 oder 4 bilden können, oder $R^3$ und $R^4$ falls Ring B mit Imidazolidindion substituiert ist, auch gemeinsam eine gegebenenfalls mit ($C_1$-$C_4$)-Alkyl substituierte $-(CH_2)_m$-Gruppe mit m = 3, 4 oder 5 bilden können,

sowie deren physiologisch verträgliche Salze.

**2.** Verbindungen der Formel I gemäß Anspruch 1, worin

a) das Chinoxalin-Ringsystem über die Position 2' oder 3' mit dem Imidazolidindion verbunden ist und $R^1$ und $R^2$ in den Positionen 6' bzw. 7' stehen und Wasserstoff, ($C_1$-$C_4$)-Alkyl, Phenyl, ($C_1$-$C_4$)-Alkoxy, Halogen, Halogen-($C_1$-$C_4$)-alkyl, Amino oder Hydroxy bedeuten, einer der Substituenten $R^3$ oder $R^4$ ein Wasserstoffatom, ($C_1$-$C_4$)-Alkyl, Phenyl, Pyridyl, Furyl, Thienyl oder Benzofuryl bedeutet und $R^5$ Wasserstoff oder ($C_1$-$C_4$)-Alkyl ist oder die Reste $R^3$ bzw. $R^4$ und $R^5$ gemeinsam eine gegebenenfalls mit ($C_1$-$C_4$)-Alkyl substituierte $-(CH_2)_n$-Gruppe mit n = 2, 3 oder 4 bilden, oder

b) das Chinoxalin-System über eine Position 5', 6' oder 7' mit dem Imidazolidindion verbunden ist, und

- $R^3$ und $R^4$ ein Wasserstoffatom, ($C_1$-$C_6$)-Alkyl, ($C_5$-$C_7$)-Cycloalkyl, Phenyl-($C_1$-$C_2$)-alkyl, Phenyl, Pyridyl, Furyl, Thienyl oder Benzofuryl bedeuten, $R^1$ ein Wasserstoffatom ist und $R^2$ ein Wasserstoffatom, ($C_1$-$C_4$)-Alkyl oder Phenyl bedeuten und $R^5$ Wasserstoff oder $C_1$-$C_4$)-Alkyl ist, oder
- die Reste $R^3$ und $R^4$ gemeinsam eine gegebenenfalls mit ($C_1$-$C_4$)-Alkyl substituierte $-(CH_2)_m$-Gruppe mit m = 3, 4 oder 5 bilden,

sowie deren physiologisch verträgliche Salze.

**3.** Verbindungen der Formel I gemäß 1, worin

a) das Chinoxalin-Ringsystem über die Position 2' oder 3' an die Imidazolidindion-Partialstruktur gebunden ist und die Substituenten $R^1$, $R^2$ in den Positionen 6' und 7' stehen und ein Wasserstoffatom, ($C_1$-$C_4$)-Alkyl, ($C_1$-$C_4$)-Alkoxy, Fluor, Trifluormethyl, Amino oder Hydroxy bedeuten und $R^3$ oder $R^4$ ein Wasserstoffatom, ($C_1$-$C_4$)-Alkyl oder Phenyl bedeutet und $R^5$ Wasserstoff oder Methyl ist oder die Reste $R^3$ und $R^5$ oder $R^4$ und $R^5$ gemeinsam eine gegebenenfalls mit ($C_1$-$C_4$)-Alkyl substituierte $-(CH_2)_3$-Gruppe bilden, oder

b) das Chinoxalin-Ringsystem über die Position 6' oder 7' an die Imidazolidindion-Partialstruktur gebunden ist und die Substituenten $R^3$, $R^4$ ein Wasserstoffatom, ($C_1$-$C_6$)-Alkyl, ($C_5$-$C_7$)-Cycloalkyl, Benzyl, Phenyl, Furyl oder Thienyl bedeuten, $R^1$ Wasserstoff ist und $R^2$ ein Wasserstoffatom, ($C_1$-$C_4$)-Alkyl oder Phenyl bedeutet und $R^5$ Wasserstoff oder Methyl ist oder die Reste $R^3$ und $R^4$ gemeinsam eine $-(CH_2)_4$-Gruppe bilden,

sowie deren physiologisch verträgliche Salze.

**4.** Verfahren zur Herstellung von Verbindungen der Formel I gemäß Anspruch 1, dadurch gekennzeichnet, daß man

a) eine Carbonylverbindung der Formel II

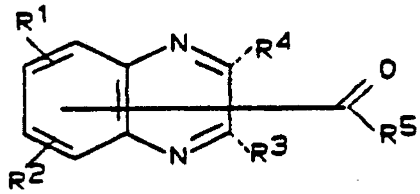

II

worin $R^1$, $R^2$, $R^3$, $R^4$ und $R^5$ die zu Formel I angegebenen Bedeutungen haben mit Cyaniden und Ammoniumcarbonat zu Verbindungen der Formel I umsetzt oder

b) eine Verbindung der Formel III

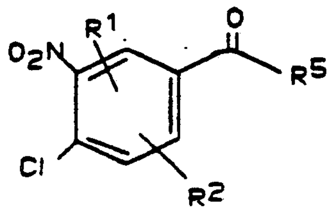

III

worin $R^1$ Wasserstoff und $R^2$ und $R^5$ die zu Formel I angegebenen Bedeutungen haben mit Benzylamin

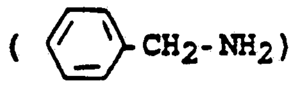

zu einer Verbindung der Formel IV

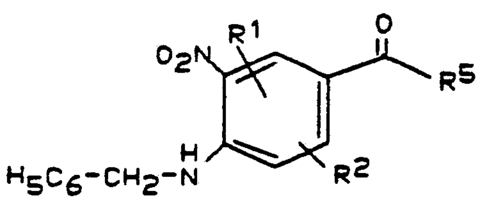

IV

umsetzt, die erhaltene Verbindung der Formel IV mit einem Cyanid, vorzugsweise einem Alkali- oder Erdalkalimetallcyanid, und Ammoniumcarbonat zu einer Verbindung der Formel V

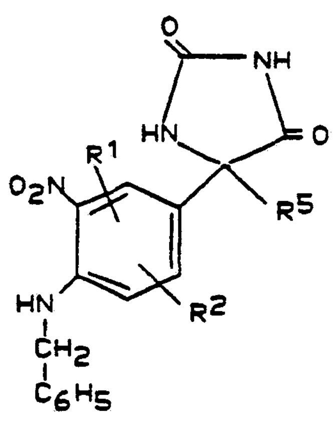

V

umsetzt, die erhaltene Verbindung in Gegenwart eines Katalysators zu einer Verbindung der Formel VI

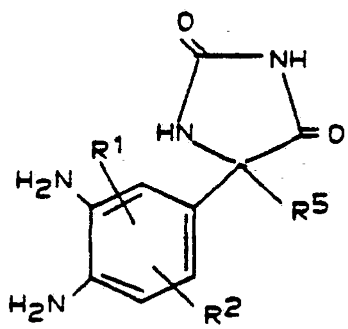

VI

reduziert und anschließend mit einer Dicarbonylverbindung VII

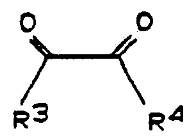

VII

zu einer Verbindung der Formel I

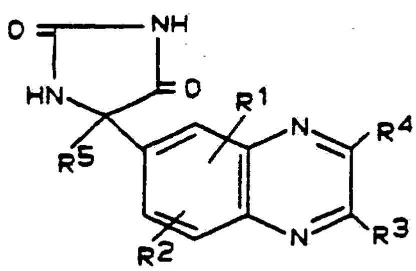

I

worin $R^1$ Wasserstoff ist und $R^2$, $R^3$, $R^4$, $R^5$ die angegebenen Bedeutungen haben, umsetzt und die Reaktionsprodukte gegebenenfalls mit Basen in physiologisch verträgliche Salze überführt.

5. Arzneimittel, dadurch gekennzeichnet, daß es eine Verbindung gemäß Anspruch 1 enthält.

6. Verfahren zur Herstellung eines Arzneimittels, dadurch gekennzeichnet, daß man eine Verbindung gemäß Anspruch 1 in eine geeignete Darreichungsform bringt.

7. Verbindungen gemäß Anspruch 1 zur Hemmung der Aldose-Reduktase.

**Patentansprüche für folgende Vertragsstaaten : ES, GR**

1. Verfahren zur Herstellung von 5-Chinoxalyl-imidazolidin-2,4-dionen der Formel I

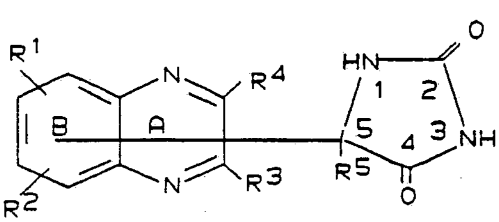

I

in welcher
- das Chinoxalin-Ringsystem über die Positionen 2', 3', 5', 6', 7', oder 8' mit dem Imidazolidindion verbunden ist,
- $R^1$ und $R^2$ gleich oder verschieden sind und Wasserstoff, $(C_1\text{-}C_6)$-Alkyl, $(C_5\text{-}C_7)$-Cycloalkyl,

Phenyl-($C_1$-$C_4$)-alkyl, Naphthyl-($C_1$-$C_4$)-alkyl, ($C_6$-$C_{12}$)-Aryl, 5- bis 6-gliedriger Heteroarylring mit Schwefel, Sauerstoff oder 1 bis 2 Stickstoffatomen als Heteroatom(e), der gegebenenfalls benzoanneliert ist, ($C_1$-$C_4$)-Alkoxy, Halogen, Halogen-($C_1$-$C_4$)-alkyl, Nitro, Amino oder Hydroxy bedeuten;

- $R^3$ und $R^4$ gleich oder verschieden sind und Wasserstoff, ($C_1$-$C_6$)-Alkyl, ($C_5$-$C_7$)-Cycloalkyl, Phenyl-($C_1$-$C_4$)-alkyl, Naphthyl-($C_1$-$C_4$)-alkyl, ($C_6$-$C_{12}$)-Aryl, 5- bis 6-gliedriger Heteroarylring mit Schwefel, Sauerstoff oder 1 bis 2 Stickstoffatomen als Heteroatom(e), der gegebenenfalls benzoanneliert ist, Amino, Nitro oder Hydroxy bedeuten;
- $R^5$ ein Wasserstoffatom , ($C_1$-$C_6$)-Alkyl, ($C_5$-$C_7$)-Cycloalkyl, Phenyl-($C_1$-$C_4$)-alkyl, Naphthyl-($C_1$-$C_4$)-alkyl oder ($C_6$-$C_{12}$)-Aryl bedeutet,
- $R^3$ und $R^5$ oder $R^4$ und $R^5$ falls Ring A mit Imidazolidindion substituiert ist, auch gemeinsam eine gegebenenfalls mit ($C_1$-$C_4$)-Alkyl substituierte $-(CH_2)_n$-Gruppe mit $n = 2, 3$ oder 4 bilden können, oder $R^3$ und $R^4$ falls Ring B mit Imidazolidindion substituiert ist, auch gemeinsam eine gegebenenfalls mit ($C_1$-$C_4$)-Alkyl substituierte $-(CH_2)_m$-Gruppe mit $m = 3, 4$ oder 5 bilden können,

sowie von deren physiologisch verträglichen Salzen, dadurch gekennzeichnet, daß man

a) eine Carbonylverbindung der Formel II

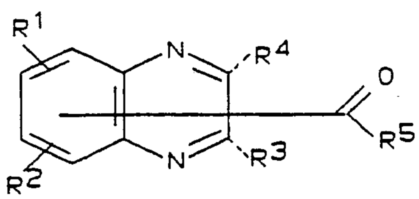

II

worin $R^1$, $R^2$, $R^3$, $R^4$ und $R^5$ die zu Formel I angegebenen Bedeutungen haben mit Cyaniden und Ammoniumcarbonat zu Verbindungen der Formel I umsetzt oder

b) eine Verbindung der Formel III

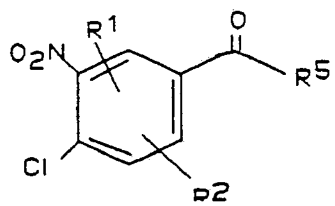

III

worin $R^1$ Wasserstoff und $R^2$ und $R^5$ die zu Formel I angegebenen Bedeutungen haben mit Benzylamin

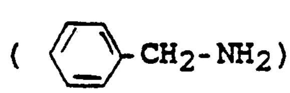

zu einer Verbindung der Formel IV

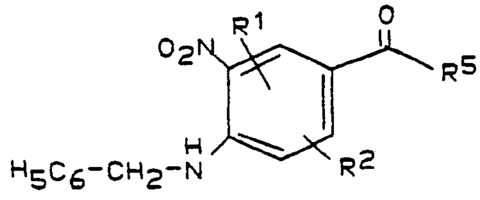

IV

umsetzt, die erhaltene Verbindung der Formel IV mit einem Cyanid, vorzugsweise einem Alkali- oder Erdalkalimetallcyanid, und Ammoniumcarbonat zu einer Verbindung der Formel V

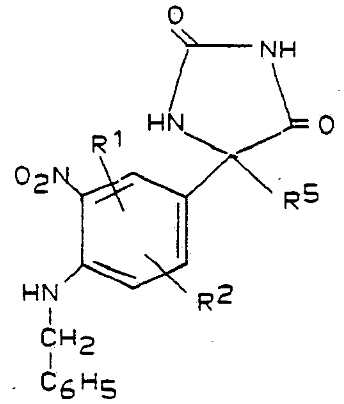

V

umsetzt, die erhaltene Verbindung in Gegenwart eines Katalysators zu einer Verbindung der Formel VI

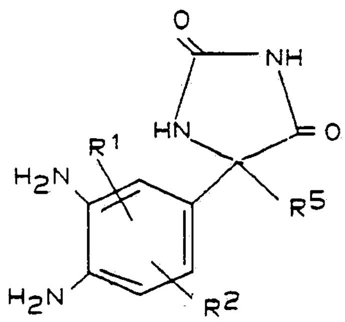

VI

reduziert und anschließend mit einer Dicarbonylverbindung VII

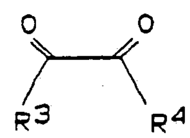

VII

zu einer Verbindung der Formel I

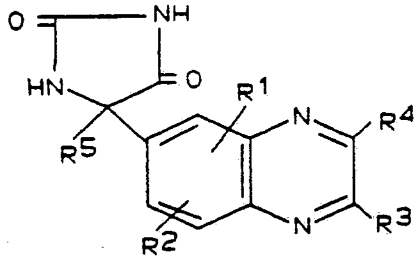

I

worin $R^1$ Wasserstoff und $R^2$, $R^3$, $R^4$, $R^5$ die angegebenen Bedeutungen haben, umsetzt und die Reaktionsprodukte gegebenenfalls mit Basen in physiologisch verträgliche Salze überführt.

**2.** Verfahren gemäß Anspruch 1, dadurch gekennzeichnet, daß Verbindungen der Formel I, worin

a) das Chinoxalin-Ringsystem über die Position 2' oder 3' mit dem Imidazolidindion verbunden ist und $R^1$ und $R^2$ in den Positionen 6' bzw. 7' stehen und Wasserstoff, ($C_1$-$C_4$)-Alkyl, Phenyl, ($C_1$-$C_4$)-Alkoxy, Halogen, Halogen-($C_1$-$C_4$)-alkyl, Amino oder Hydroxy bedeuten, einer der Substituenten $R^3$ oder $R^4$ ein Wasserstoffatom, ($C_1$-$C_4$)-Alkyl, Phenyl, Pyridyl, Furyl, Thienyl oder Benzofuryl bedeutet und $R^5$ Wasserstoff oder ($C_1$-$C_4$)-Alkyl ist oder die Reste $R^3$ bzw. $R^4$ und $R^5$ gemeinsam eine

gegebenenfalls mit ($C_1$-$C_4$)-Alkyl substituierte -$(CH_2)_n$-Gruppe mit n = 2, 3 oder 4 bilden, oder

b) das Chinoxalin-System über eine Position 5', 6' oder 7' mit dem Imidazolidindion verbunden ist, und

- $R^3$ und $R^4$ ein Wasserstoffatom, ($C_1$-$C_6$)-Alkyl, ($C_5$-$C_7$)-Cycloalkyl, Phenyl-($C_1$-$C_2$)-alkyl, Phenyl, Pyridyl, Furyl, Thienyl oder Benzofuryl bedeuten, $R^1$ ein Wasserstoffatom ist und $R^2$ ein Wasserstoffatom, ($C_1$-$C_4$)-Alkyl oder Phenyl bedeuten und $R^5$ Wasserstoff oder $C_1$-$C_4$)-Alkyl ist, oder
- die Reste $R^3$ und $R^4$ gemeinsam eine gegebenenfalls mit ($C_1$-$C_4$)-Alkyl substituierte -$(CH_2)_m$-Gruppe mit m = 3, 4 oder 5 bilden,

sowie deren physiologisch verträgliche Salze herstellt.

3. Verfahren gemäß Anspruch 1, dadurch gekennzeichnet, daß man Verbindungen der Formel I, worin

a) das Chinoxalin-Ringsystem über die Position 2' oder 3' an die Imidazolidindion-Partialstruktur gebunden ist und die Substituenten $R^1$, $R^2$ in den Positionen 6' und 7' stehen und ein Wasserstoffatom, ($C_1$-$C_4$)-Alkyl, ($C_1$-$C_4$)-Alkoxy, Fluor, Trifluormethyl, Amino oder Hydroxy bedeuten und $R^3$ oder $R^4$ ein Wasserstoffatom, ($C_1$-$C_4$)-Alkyl oder Phenyl bedeutet und $R^5$ Wasserstoff oder Methyl ist oder die Reste $R^3$ und $R^5$ oder $R^4$ und $R^5$ gemeinsam eine gegebenenfalls mit ($C_1$-$C_4$)-Alkyl substituierte -$(CH_2)_3$-Gruppe bilden, oder

b) das Chinoxalin-Ringsystem über die Position 6' oder 7' an die Imidazolidindion-Partialstruktur gebunden ist und die Substituenten $R^3$, $R^4$ ein Wasserstoffatom, ($C_1$-$C_6$)-Alkyl, ($C_5$-$C_7$)-Cycloalkyl, Benzyl, Phenyl, Furyl oder Thienyl bedeuten, $R^1$ Wasserstoff ist und $R^2$ ein Wasserstoffatom, ($C_1$-$C_4$)-Alkyl oder Phenyl bedeutet und $R^5$ Wasserstoff oder Methyl ist oder die Reste $R^3$ und $R^4$ gemeinsam eine -$(CH_2)_4$-Gruppe bilden,

sowie deren physiologisch verträgliche Salze herstellt.

4. Verfahren zur Herstellung eines Arzneimittels, dadurch gekennzeichnet, daß man eine Verbindung gemäß Anspruch 1 in eine geeignete Darreichungsform bringt.

**Claims**

**Claims for the following Contracting States : AT, BE, CH, DE, FR, GR, IT, LI, LU, NL, SE**

1. A 5-quinoxalyl-imidazolidine-2,4-dione of the formula I

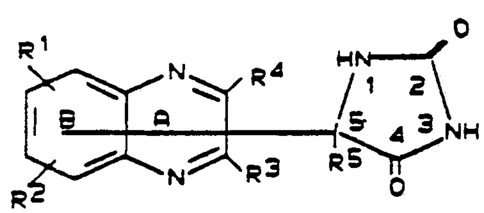

I

in which

- the quinoxaline ring system is bonded to the imidazolidinedione via positions 2', 3', 5', 6', 7' or 8',
- $R^1$ and $R^2$ are identical or different and denote hydrogen, ($C_1$-$C_8$)-alkyl, ($C_5$-$C_7$)-cycloalkyl, phenyl-($C_1$-$C_4$)-alkyl, naphthyl-($C_1$-$C_4$)-alkyl, ($C_6$-$C_{12}$)-aryl, a 5- or 6-membered heteroaryl ring which has sulfur, oxygen or 1 or 2 nitrogen atoms as hetero atom(s) and is optionally benzo-fused, ($C_1$-$C_4$)-alkoxy, halogen, halogeno-($C_1$-$C_4$)-alkyl, nitro, amino or hydroxyl;
- $R^3$ and $R^4$ are identical or different and denote hydrogen, ($C_1$-$C_6$)-alkyl, ($C_5$-$C_7$)-cycloalkyl, phenyl-($C_1$-$C_4$)-alkyl, naphthyl-($C_1$-$C_4$)-alkyl, ($C_6$-$C_{12}$)-aryl, a 5- or 6-membered heteroaryl ring which has sulfur, oxygen or 1 or 2 nitrogen atoms as hetero atom(s) and is optionally benzo-fused, amino, nitro or hydroxyl;
- $R^5$ denotes a hydrogen atom, ($C_1$-$C_6$)-alkyl, ($C_5$-$C_7$)-cycloalkyl, phenyl- ($C_1$-$C_4$)-alkyl, naphthyl-($C_1$-$C_4$)-alkyl or ($C_6$-$C_{12}$)-aryl,
- $R^3$ and $R^5$ or $R^4$ and $R^5$, if ring A is substituted by imidazolidinedione, can also together form a -$(CH_2)_n$-group, where n = 2, 3 or 4, which is optionally substituted by ($C_1$-$C_4$)-alkyl, or, if ring B is substituted by imidazolidinedione, $R^3$ and $R^4$ can also together form a -$(CH_2)_m$- group, where m = 3, 4 or 5, which is optionally substituted by ($C_1$-$C_4$)-alkyl,

or a physiologically tolerated salt thereof.

**2.** A compound of the formula I as claimed in claim 1, in which

a) the quinoxaline ring system is bonded to the imidazolidinedione via position 2' or 3' and $R^1$ and $R^2$ are in positions 6' and 7' respectively and denote hydrogen, $(C_1-C_4)$-alkyl, phenyl, $(C_1-C_4)$-alkoxy, halogen, halogeno-$(C_1-C_4)$-alkyl, amino or hydroxyl, one of the substituents $R^3$ or $R^4$ denotes a hydrogen atom, $(C_1-C_4)$-alkyl, phenyl, pyridyl, furyl, thienyl or benzofuryl and $R^5$ is hydrogen or $(C_1-C_4)$-alkyl, or the radicals, $R^3$ or $R^4$ and $R^5$ together form a $-(CH_2)_n$-group, where n = 2, 3 or 4, which is optionally substituted by $(C_1-C_4)$-alkyl, or

b) the quinoxaline system is bonded to the imidazolidinedione via position 5', 6' or 7' and

- $R^3$ and $R^4$ denote a hydrogen atom, $(C_1-C_6)$-alkyl, $(C_5-C_7)$-cycloalkyl, phenyl-$(C_1-C_2)$-alkyl, phenyl, pyridyl, furyl, thienyl or benzofuryl, $R^1$ is a hydrogen atom, $R^2$ is a hydrogen atom, $(C_1-C_4)$-alkyl or phenyl and $R^5$ is hydrogen or $(C_1-C_4)$-alkyl, or
- the radicals $R^3$ and $R^4$ together form a $-(CH_2)_m-$ group, where m = 3, 4 or 5, which is optionally substituted by $(C_1-C_4)$-alkyl,

or a physiologically tolerated salt thereof.

**3.** A compound of the formula I as claimed in claim 1, in which

a) the quinoxaline ring system is bonded to the imidazolidinedione partial structure via position 2' or 3' and the substituents $R^1$ and $R^2$ are in positions 6' and 7' and denote a hydrogen atom, $(C_1-C_4)$-alkyl, $(C_1-C_4)$-alkoxy, fluorine, trifluoromethyl, amino or hydroxyl, $R^3$ or $R^4$ denotes a hydrogen atom, $(C_1-C_4)$-alkyl or phenyl and $R^5$ is hydrogen or methyl, or the radicals $R^3$ and $R^5$ or $R^4$ and $R^5$ together form a $-(CH_2)_3$-group which is optionally substituted by $(C_1-C_4)$-alkyl, or

b) the quinoxaline ring system is bonded to the imidazolidinedione partial structure via position 6' or 7' and the substituents $R^3$ and $R^4$ denote a hydrogen atom, $(C_1-C_6)$-alkyl, $(C_5-C_7)$-cycloalkyl, benzyl, phenyl, furyl or thienyl, $R^1$ is hydrogen, $R^2$ denotes a hydrogen atom, $(C_1-C_4)$-alkyl or phenyl and $R^5$ is hydrogen or methyl, or the radicals $R^3$ and $R^4$ together form a $-(CH_2)_4-$ group,

or a physiologically tolerated salt thereof.

**4.** A process for the preparation of a compound of the formula I as claimed in claim 1, which comprises

a) reacting a carbonyl compound of the formula II

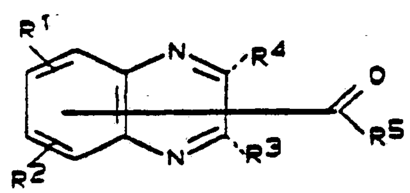

II

in which $R^1$, $R^2$, $R^3$, $R^4$ and $R^5$ have the meanings given in the case of formula I, with a cyanide and ammonium carbonate to give a compound of the formula I, or

b) reacting a compound of the formula III

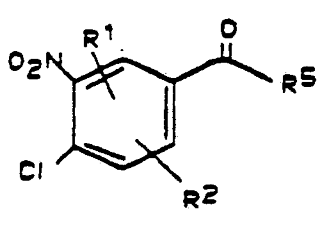

III

in which $R^1$ is hydrogen and $R^2$ and $R^5$ have the meanings given in the case of formula I, with benzylamine

( 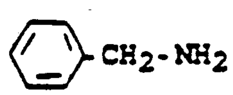 )

to give a compound of the formula IV

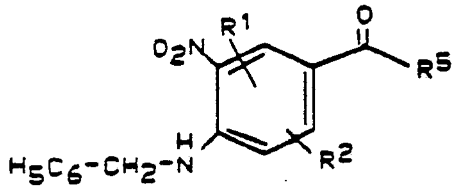

IV

reacting the resulting compound of the formula IV with a cyanide, preferably an alkali metal or alkaline earth metal cyanide, and ammonium carbonate to give a compound of the formula V

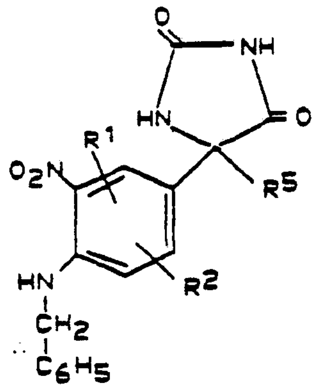

V

reducing the resulting compound in the presence of a catalyst to give a compound of the formula VI

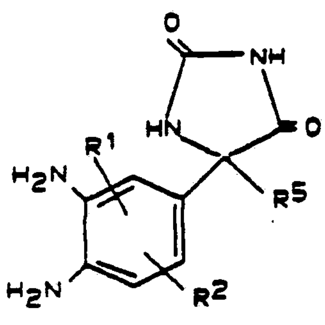

VI

and subsequently reacting this with a dicarbonyl compound VII

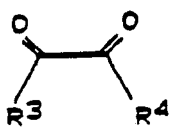

VII

to give a compound of the formula I

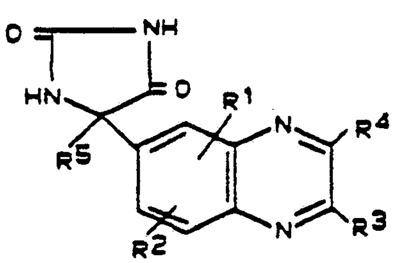

I

in which $R^1$ denotes hydrogen and $R^2$, $R^3$, $R^4$ and $R^5$ have the meanings given, and, if appropriate,

converting the reaction product into a physiologically tolerated salt using a base.

**5.** A medicament which contains a compound as claimed in claim 1.

**6.** A process for the preparation of a medicament, which comprises bringing a compound as claimed in claim 1 into a suitable formulation.

**7.** A compound as claimed in claim 1 for inhibiting aldose reductase.

**Claims for the following Contracting States : ES, GR**

**1.** A process for the preparation of a 5-quinoxalyl-imidazolidine-2,4-dione of the formula I

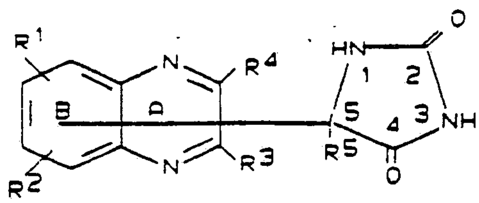

I

in which

- the quinoxaline ring system is bonded to the imidazolidinedione via positions 2', 3', 5', 6', 7' or 8',
- $R^1$ and $R^2$ are identical or different and denote hydrogen, ($C_1$-$C_6$)-alkyl, ($C_5$-$C_7$)-cycloalkyl, phenyl-($C_1$-$C_4$)-alkyl, naphthyl-($C_1$-$C_4$)-alkyl, ($C_6$-$C_{12}$)-aryl, a 5- or 6-membered heteroaryl ring which has sulfur, oxygen or 1 or 2 nitrogen atoms as hetero atom(s) and is optionally benzo-fused, ($C_1$-$C_4$)-alkoxy, halogen, halogeno-($C_1$-$C_4$)-alkyl, nitro, amino or hydroxyl;
- $R^3$ and $R^4$ are identical or different and denote hydrogen, ($C_1$-$C_6$)-alkyl, ($C_5$-$C_7$)-cycloalkyl, phenyl-($C_1$-$C_4$)-alkyl, naphthyl-($C_1$-$C_4$)-alkyl, ($C_6$-$C_{12}$)-aryl, a 5- or 6-membered heteroaryl ring which has sulfur, oxygen or 1 or 2 nitrogen atoms as hetero atom(s) and is optionally benzo-fused, amino, nitro or hydroxyl;
- $R^5$ denotes a hydrogen atom, ($C_1$-$C_6$)-alkyl, ($C_5$-$C_7$)-cycloalkyl, phenyl-($C_1$-$C_4$)-alkyl, naphthyl-($C_1$-$C_4$)-alkyl or ($C_6$-$C_{12}$)-aryl,
- $R^3$ and $R^5$ or $R^4$ and $R^5$, if ring A is substituted by imidazolidinedione, can also together form a $-(CH_2)_n$-group, where n = 2, 3 or 4, which is optionally substituted by ($C_1$-$C_4$)-alkyl, or, if ring B is substituted by imidazolidinedione, $R^3$ and $R^4$ can also together form a $-(CH_2)_m-$ group, where m = 3, 4 or 5, which is optionally substituted by ($C_1$-$C_4$)-alkyl,

or a physiologically tolerated salt thereof, which comprises

a) reacting a carbonyl compound of the formula II

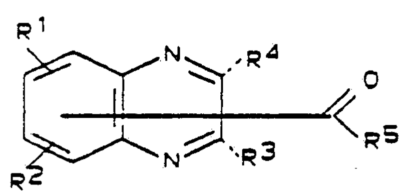

II

in which $R^1$, $R^2$, $R^3$, $R^4$ and $R^5$ have the meanings given in the case of formula I, with a cyanide and ammonium carbonate to give a compound of the formula I, or

b) reacting a compound of the formula III

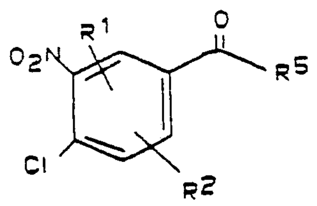

III

in which $R^1$ is hydrogen and $R^2$ and $R^3$ have the meanings given in the case of formula I, with benzylamine

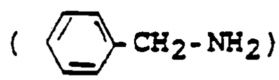

to give a compound of the formula IV

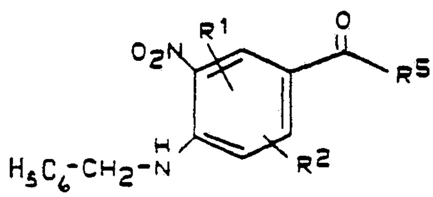

IV

reacting the resulting compound of the formula IV with a cyanide, preferably an alkali metal or alkaline earth metal cyanide, and ammonium carbonate to give a compound of the formula V

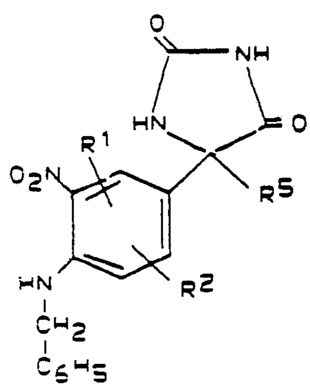

V

reducing the resulting compound in the presence of a catalyst to give a compound of the formula VI

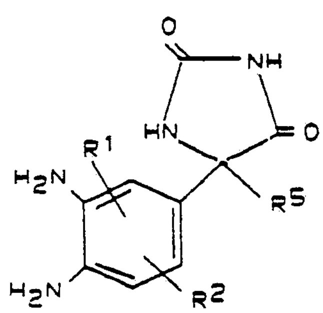

VI

and subsequently reacting this with a dicarbonyl compound VII

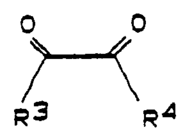

VII

to give a compound of the formula I

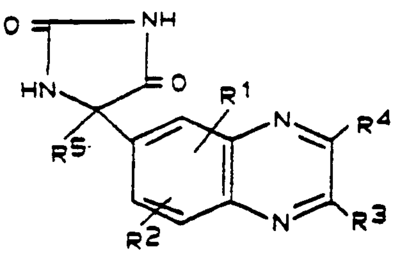

I

in which $R^1$ denotes hydrogen and $R^2$, $R^3$, $R^4$ and $R^5$ have the meanings given, and, if appropriate, converting the reaction product into a physiologically tolerated salt using a base.

**2.** The process as claimed in claim 1, wherein a compound of the formula I, in which
a) the quinoxaline ring system is bonded to the imidazolidinedione via position 2' or 3' and $R^1$ and $R^2$ are in positions 6' and 7' respectively and denote hydrogen, $(C_1-C_4)$-alkyl, phenyl, $(C_1-C_4)$-alkoxy, halogen, halogeno-$(C_1-C_4)$-alkyl, amino or hydroxyl, one of the substituents $R^3$ or $R^4$ denotes a hydrogen atom, $(C_1-C_4)$-alkyl, phenyl, pyridyl, furyl, thienyl or benzofuryl and $R^5$ is hydrogen or $(C_1-C_4)$-alkyl, or the radicals, $R^3$ or $R^4$ and $R^5$ together form a $-(CH_2)_n$-group, where n = 2, 3 or 4, which is optionally substituted by $(C_1-C_4)$-alkyl, or
b) the quinoxaline system is bonded to the imidazolidinedione via position 5', 6' or 7' and
- $R^3$ and $R^4$ denote a hydrogen atom, $(C_1-C_6)$-alkyl, $(C_5-C_7)$-cycloalkyl, phenyl-$(C_1-C_2)$-alkyl, phenyl, pyridyl, furyl, thienyl or benzofuryl, $R^1$ is a hydrogen atom, $R^2$ is a hydrogen atom, $(C_1-C_4)$-alkyl or phenyl and $R^5$ is hydrogen or $(C_1-C_4)$-alkyl, or
- the radicals $R^3$ and $R^4$ together form a $-(CH_2)_m-$ group, where m = 3, 4 or 5, which is optionally substituted by $(C_1-C_4)$-alkyl,

or a physiologically tolerated salt thereof is prepared.

**3.** The process as claimed in claim 1, wherein a compound of the formula I, in which
a) the quinoxaline ring system is bonded to the imidazolidinedione partial structure via position 2' or 3' and the substituents $R^1$ and $R^2$ are in positions 6' and 7' and denote a hydrogen atom, $(C_1-C_4)$-alkyl, $(C_1-C_4)$-alkoxy, fluorine, trifluoromethyl, amino or hydroxyl, $R^3$ or $R^4$ denotes a hydrogen atom, $(C_1-C_4)$-alkyl or phenyl and $R^5$ is hydrogen or methyl, or the radicals $R^3$ and $R^5$ or $R^4$ and $R^5$ together form a $-(CH_2)_3$-group which is optionally substituted by $(C_1-C_4)$-alkyl, or
b) the quinoxaline ring system is bonded to the imidazolidinedione partial structure via position 6' or 7' and the substituents $R^3$ and $R^4$ denote a hydrogen atom, $(C_1-C_6)$-alkyl, $(C_5-C_7)$-cycloalkyl, benzyl, phenyl, furyl or thienyl, $R^1$ is hydrogen, $R^2$ denotes a hydrogen atom, $(C_1-C_4)$-alkyl or phenyl and $R^5$ is hydrogen or methyl, or the radicals $R^3$ and $R^4$ together form a $-(CH_2)_4$-group,
or a physiologically tolerated salt thereof is prepared.

**4.** A process for the preparation of a medicament, which comprises bringing a compound as claimed in claim 1 into a suitable formulation.

**Revendications**
**Revendications pour les Etats contractants suivants : AT, BE, CH, DE, FR, GB, IT, LI, LU, NL, SE**

**1.** 5-quinoxalyl-imidazolidine-2,4-diones de formule I

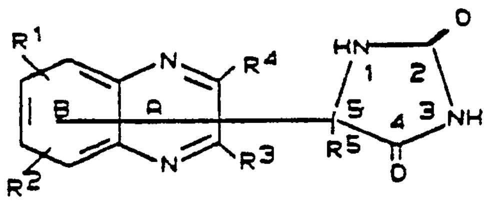

I

dans laquelle

- le système cyclique quinoxaline est lié par les positions 2', 3', 5', 6', 7' ou 8' à l'imidazolidine-dione;
- $R^1$ et $R^2$ sont identiques ou différents et représentent des atomes d'hydrogène, des groupes alkyle en $C_1$-$C_6$, cycloalkyle en $C_5$-$C_7$, phényl-alkyle($C_1$-$C_4$), naphtyl-alkyle($C_1$-$C_4$), aryle en $C_6$-$C_{12}$, un cycle hétéroaryle à 5 ou 6 chaînons comportant un atome de soufre, d'oxygène ou un ou deux atomes d'azote en tant qu'hétéroatome(s), qui est éventuellement soudé à un noyau benzénique, des atomes d'halogène, des groupes alcoxy en $C_1$-$C_4$, halogénoalkyle en $C_1$-$C_4$, nitro, amino ou hydroxy;
- $R^3$ et $R^4$ sont identiques ou différents et représentent des atomes d'hydrogène, des groupes alkyle en $C_1$-$c_6$, cycloalkyle en $C_5$-$C_7$, phényl-alkyle($C_1$-$C_4$), naphtyl-alkyle($C_1$-$C_4$), aryle en $C_6$-$c_{12}$, un cycle hétéroaryle à 5 ou 6 chaînons comportant des atomes de soufre, oxygène ou un ou deux atomes d'azote en tant qu'hétéroatome(s), qui est éventuellement soudé à un noyau benzénique, le groupe amino, nitro ou hydroxy;
- $R^5$ représente un atome d'hydrogène, un groupe alkyle en $C_1$-$C_6$, cycloalkyle en $C_5$-$C_7$, phényl-alkyle($C_1$-$C_4$), naphtyl-alkyle($C_1$-$C_4$) ou aryle en $C_6$-$C_{12}$;
- $R^3$ et $R^5$ ou $R^4$ et $R^5$, dans le cas où le cycle A est substitué par l'imidazolidine-dione, peuvent également former ensemble un groupe $-(CH_2)_n-$, avec n = 2, 3 ou 4, éventuellement substitué par un radical alkyle en $C_1$-$C_4$, ou $R^3$ et $R^4$, au cas où le cycle B est substitué par l'imidazolidine-dione, peuvent également former ensemble un groupe $-(CH_2)_m-$, avec m = 3, 4 ou 5, éventuellement substitué par un radical alkyle en $C_1$-$C_4$;

et leurs sels physiologiquement acceptables.

**2.** Composés de formule I selon la revendication 1, dans lesquels

a) le système cyclique quinoxaline est lié par la position 2' ou 3' à l'imidazolidine-dione, et $R^1$ et $R^2$ sont en positions 6' et respectivement 7' et représentent un atome d'hydrogène ou d'halogène ou un groupe alkyle en $C_1$-$C_4$,phényle, alcoxy en $C_1$-$C_4$, halogénoalkyle($C_1$-$C_4$), amino ou hydroxy, l'un des substituants $R^3$ ou $R^4$ représente un atome d'hydrogène ou un groupe alkyle en $C_1$-$C_4$, phényle, pyridyle, furyle, thiényle ou benzofuryle, et $R^5$ est un atome d'hydrogène ou un groupe alkyle en $C_1$-$C_4$, ou les radicaux $R^3$ ou $R^4$ et $R^5$ forment ensemble un groupe $-(CH_2)n-$, avec n = 2, 3 ou 4, éventuellement substitué par un radical alkyle en $C_1$-$C_4$;

b) le système quinoxaline est lié par une position 5', 6' ou 7' à l'imidazolidine-dione, et

- $R^3$ et $R^4$ représentent un atome d'hydrogène ou un groupe alkyle en $C_1$-$C_6$, cycloalkyle en $C_5$-$C_7$, phényl-alkyle($C_1$-$C_2$), phényle, pyridyle, furyle, thiényle ou benzofuryle, $R^1$ est un atome d'hydrogène et $R^2$ représente un atome d'hydrogène ou un groupe alkyle en $C_1$-$C_4$ ou phényle, et $R^5$ est un atome d'hydrogène ou un groupe alkyle en $C_1$-$C_4$, ou
- les radicaux $R^3$ et $R^4$ forment ensemble un groupe $-(CH_2)_m-$, avec m = 3, 4 ou 5, éventuellement substitué par un radical alkyle en $C_1$-$C_4$,

et sels physiologiquement acceptables de ceux-ci.

**3.** Composés de formule I selon la revendication 1, dans lesquels

a) le système cyclique quinoxaline est lié par la position 2' ou 3' à la structure partielle imidazolidine-dione et les substituants $R^1$, $R^2$ se situent dans les positions 6' et 7' et représentent un atome d'hydrogène ou de fluor ou un groupe alkyle en $C_1$-$C_4$, alcoxy en $C_1$-$C_4$, trifluorométhyle, amino ou hydroxy, et $R^3$ ou $R^4$ représentent un atome d'hydrogène ou un groupe alkyle en $C_1$ -$C_4$ ou phényle, et $R^5$ est un atome d'hydrogène ou le groupe méthyle, ou les radicaux $R^3$ et $R^5$ ou $R^4$ et $R^5$ forment ensemble un groupe $-(CH_2)_3-$ éventuellement substitué par un radical alkyle en $C_1$-$C_4$, ou

b) le système cyclique quinoxaline est lié par la position 6' ou 7' à la structure partielle

imidazolidine-dione et les substituants $R^3$, $R^4$ représentent un atome d'hydrogène ou un groupe alkyle en $C_1$-$C_6$, cycloalkyle en $C_5$-$C_7$, benzyle, phényle, furyle ou thiényle, $R^1$ est un atome d'hydrogène et $R^2$ représente un atome d'hydrogène ou un groupe alkyle en $C_1$-$C_4$ ou phényle, et $R^5$ est un atome d'hydrogène ou le groupe méthyle, ou les radicaux $R^3$ et $R^4$ forment ensemble un groupe $-(CH_2)_4-$,
et sels physiologiquement acceptables de ceux-ci.

**4.** Procédé pour la préparation des composés de formule I selon la revendication 1, caractérisé en ce que
a) on fait réagir un composé carbonyle de formule II

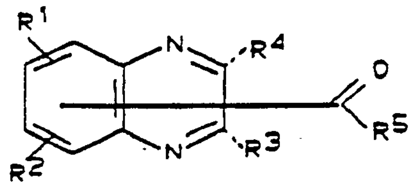

II

dans laquelle $R^1$, $R^2$, $R^3$, $R^4$ et $R^5$ ont les significations données à propos de la formule I, avec des cyanures et du carbonate d'ammonium, pour aboutir aux composés de formule I, ou
b) on fait réagir un composé de formule III

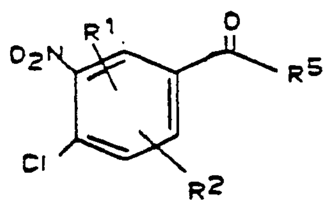

III

dans laquelle $R^1$ est un atome d'hydrogène et $R^2$ et $R^5$ ont les significations données à propos de la formule I, avec la benzylamine

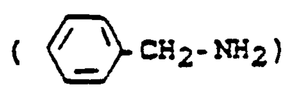

pour aboutir à un composé de formule IV

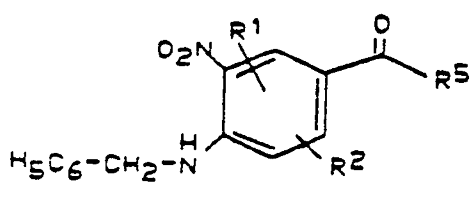

IV

on fait réagir le composé de formule IV obtenu avec un cyanure, de préférence un cyanure de métal alcalin ou alcalino-terreux, et du carbonate d'ammonium, pour aboutir à un composé de formule V

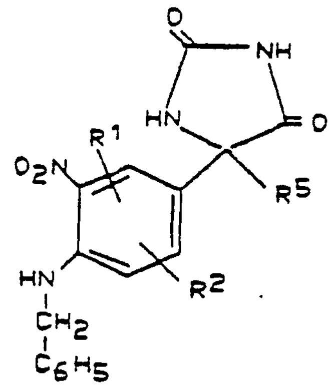

V

on réduit le composé obtenu, en présence d'un catalyseur, pour aboutir à un composé de formule VI

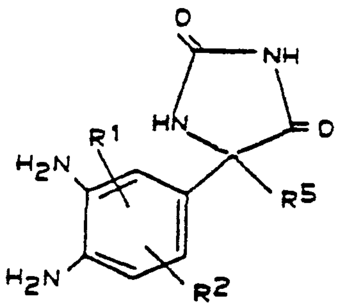

VI

et on le fait réagir ensuite avec un composé dicarbonyle VII

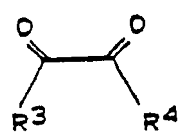

VII

pour aboutir à un composé de formule I

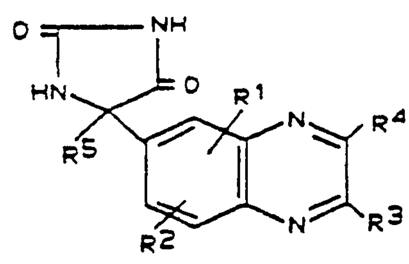

I

dans laquelle $R^1$ est un atome d'hydrogène et $R^2$, $R^3$, $R^4$ et $R^5$ ont les significations indiquées, et éventuellement on convertit à l'aide de bases les produits de réaction en sels physiologiquement acceptables.

**5.** Médicament caractérisé en ce qu'il contient un composé selon la revendication 1.

**6.** Procédé pour la fabrication d'un médicament, caractérisé en ce que l'on met sous une forme pharmaceutique appropriée un composé selon la revendication 1.

**7.** Composés selon la revendication 1 pour l'inhibition de l'aldose réductase.

**Revendications pour les Etats contractants suivants : ES, GR**

1. Procédé pour la préparation de 5-quinoxalyl-imidazolidine-2,4-diones de formule I

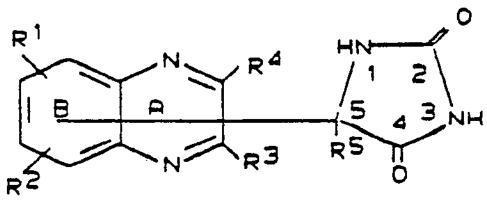

I

dans laquelle

- le système cyclique quinoxaline est lié par les positions 2', 3', 5', 6', 7' ou 8' à l'imidazolidine-dione;
- $R^1$ et $R^2$ sont identiques ou différents et représentent des atomes d'hydrogène, des groupes alkyle en $C_1$-$C_6$, cycloalkyle en $C_5$-$C_7$, phényl-alkyle($C_1$-$C_4$), naphtyl-alkyle($C_1$-$C_4$), aryle en $C_6$-$C_{12}$, un cycle hétéroaryle à 5 ou 6 chaînons comportant un atome de soufre, d'oxygène ou un ou deux atomes d'azote en tant qu'hétéroatome(s), qui est éventuellement soudé à un noyau benzénique, des atomes d'halogène, des groupes alcoxy en $C_1$-$C_4$, halogénoalkyle en $C_1$-$C_4$, nitro, amino ou hydroxy;
- $R^3$ et $R^4$ sont identiques ou différents et représentent des atomes d'hydrogène, des groupes alkyle en $C_1$-$C_6$, cycloalkyle en $C_5$-$C_7$, phényl-alkyle($C_1$-$C_4$), naphtyl-alkyle($C_1$-$C_4$), aryle en $C_6$-$C_{12}$, un cycle hétéroaryle à 5 ou 6 chaînons comportant des atomes de soufre, oxygène ou un ou deux atomes d'azote en tant qu'hétéroatome(s), qui est éventuellement soudé à un noyau benzénique, le groupe amino, nitro ou hydroxy;
- $R^5$ représente un atome d'hydrogène, un groupe alkyle en $C_1$-$C_6$, cycloalkyle en $C_5$-$C_7$, phényl-alkyle($C_1$-$C_4$), naphtyl-alkyle($C_1$-$C_4$) ou aryle en $C_6$-$C_{12}$;
- $R^3$ et $R^5$ ou $R^4$ et $R^5$, dans le cas où le cycle A est substitué par l'imidazolidine-dione, peuvent également former ensemble un groupe $-(CH_2)_n-$, avec n = 2, 3 ou 4, éventuellement substitué par un radical alkyle en $C_1$-$C_4$, ou $R^3$ et $R^4$, au cas où le cycle B est substitué par l'imidazolidine-dione, peuvent également former ensemble un groupe $-(CH_2)_m-$, avec m = 3, 4 ou 5, éventuellement substitué par un radical alkyle en $C_1$-$C_4$;

ainsi que de leurs sels physiologiquement acceptables, caractérisé en ce que

a) on fait réagir un composé carbonyle de formule II

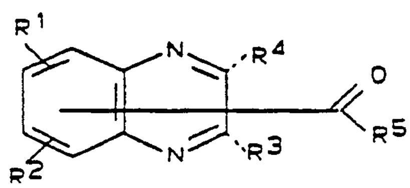

II

dans laquelle $R^1$, $R^2$, $R^3$, $R^4$ et $R^5$ ont les significations données à propos de la formule I, avec des cyanures et du carbonate d'ammonium, pour aboutir aux composés de formule I, ou

b) on fait réagir un composé de formule III

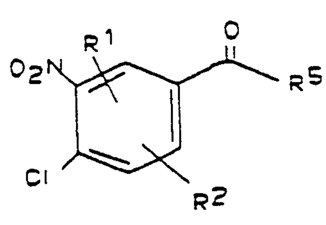

III

dans laquelle $R^1$ est un atome d'hydrogène et $R^2$ et $R^5$ ont les significations données à propos de la formule I, avec la benzylamine

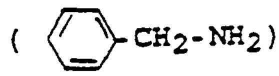

pour aboutir à un composé de formule IV

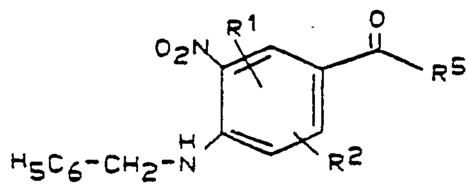

IV

on fait réagir le composé de formule IV obtenu avec un cyanure, de préférence un cyanure de métal alcalin ou alcalino-terreux, et du carbonate d'ammonium, pour aboutir à un composé de formule V

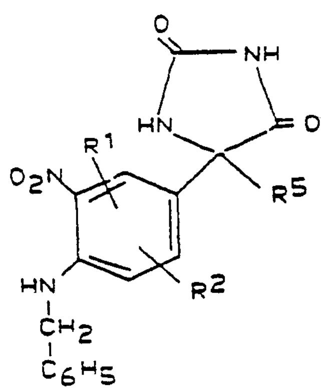

V

on réduit le composé obtenu, en présence d'un catalyseur, pour aboutir à un composé de formule VI

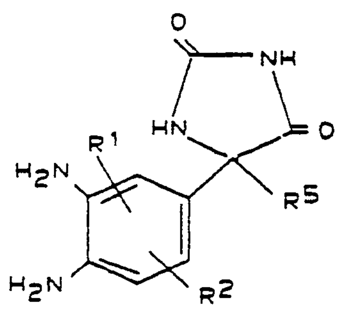

VI

et on le fait réagir ensuite avec un composé dicarbonyle VII

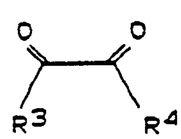

VII

pour aboutir à un composé de formule I

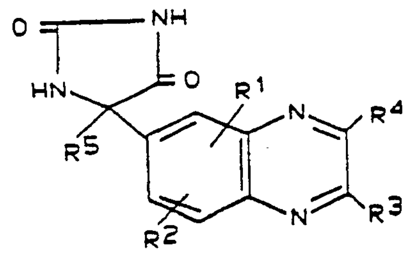

I

dans laquelle $R^1$ est un atome d'hydrogène et $R^2$, $R^3$, $R^4$ et $R^5$ ont les significations indiquées, et éventuellement on convertit à l'aide de bases les produits de réaction en sels physiologiquement acceptables.

**2.** Procédé selon la revendication 1, caractérisé en ce que l'on prépare des composés de formule I dans lesquels

a) le système cyclique quinoxaline est lié par la position 2' ou 3' à l'imidazolidine-dione, et $R^1$ et $R^2$ sont en positions 6' et respectivement 7' et représentent un atome d'hydrogène ou d'halogène ou un groupe alkyle en $C_1$-$C_4$,phényle, alcoxy en $C_1$-$C_4$, halogénoalkyle($C_1$-$C_4$), amino ou hydroxy, l'un des substituants $R^3$ ou $R^4$ représente un atome d'hydrogène ou un groupe alkyle en $C_1$-$C_4$, phényle, pyridyle, furyle, thiényle ou benzofuryle, et $R^5$ est un atome d'hydrogène ou un groupe alkyle en $C_1$-$C_4$, ou les radicaux $R^3$ ou $R^4$ et $R^5$ forment ensemble un groupe $-(CH_2)n-$, avec n = 2, 3 ou 4, éventuellement substitué par un radical alkyle en $C_1$-$C_4$;

b) le système quinoxaline est lié par une position 5', 6' ou 7' à l'imidazolidine-dione, et

- $R^3$ et $R^4$ représentent un atome d'hydrogène ou un groupe alkyle en $C_1$-$C_6$, cycloalkyle en $C_5$-$C_7$, phényl-alkyle($C_1$-$C_2$), phényle, pyridyle, furyle, thiényle ou benzofuryle, $R^1$ est un atome d'hydrogène et $R^2$ représente un atome d'hydrogène ou un groupe alkyle en $C_1$-$C_4$ ou phényle, et $R^5$ est un atome d'hydrogène ou un groupe alkyle en $C_1$-$C_4$, ou
- les radicaux $R^3$ et $R^4$ forment ensemble un groupe $-(CH_2)_m-$, avec m = 3, 4 ou 5, éventuellement substitué par un radical alkyle en $C_1$-$C_4$,

ainsi que de leurs sels physiologiquement acceptables.

**3.** Procédé selon la revendication 1, caractérisé en ce que l'on prépare des composés de formule I dans lesquels

a) le système cyclique quinoxaline est lié par la position 2' ou 3' à la structure partielle imidazolidine-dione et les substituants $R^1$, $R^2$ se situent dans les positions 6' et 7' et représentent un atome d'hydrogène ou de fluor ou un groupe alkyle en $C_1$-$C_4$, alcoxy en $C_1$-$C_4$, trifluorométhyle, amino ou hydroxy, et $R^3$ ou $R^4$ représentent un atome d'hydrogène ou un groupe alkyle en $C_1$-$C_4$ ou phényle, et $R^5$ est un atome d'hydrogène ou le groupe méthyle, ou les radicaux $R^3$ et $R^5$ ou $R^4$ et $R^5$ forment ensemble un groupe $-(CH_2)_3-$ éventuellement substitué par un radical alkyle en $C_1$-$C_4$, ou

b) le système cyclique quinoxaline est lié par la position 6' ou 7' à la structure partielle imidazolidine-dione et les substituants $R^3$, $R^4$ représentent un atome d'hydrogène ou un groupe alkyle en $C_1$-$C_6$, cycloalkyle en $C_5$-$C_7$, benzyle, phényle, furyle ou thiényle, $R^1$ est un atome d'hydrogène et $R^2$ représente un atome d'hydrogène ou un groupe alkyle en $C_1$-$C_4$ ou phényle, et $R^5$ est un atome d'hydrogène ou le groupe méthyle, ou les radicaux $R^3$ et $R^4$ forment ensemble un groupe $-(CH_2)_4-$,

ainsi que de leurs sels physiologiquement acceptables.

**4.** Procédé pour la fabrication d'un médicament, caractérisé en ce que l'on met sous une forme pharmaceutique appropriée un composé selon la revendication 1.